# EUROPEAN PATENT APPLICATION

(11) **EP 2 256 492 A1**
(43) Date of publication of application: **01.12.2010**
(21) Application number: 09161147.5
(22) Date of filing: 26.05.2009
(51) Int. Cl.: G01N 33/487

(54) **Method for the ex vivo analysis of a cell and apparatus therefore**

(71) Applicant: Stichting Katholieke Universiteit, Radboud Universiteit Nijmegen, 6525 HP Nijmegen (NL)
(72) Inventor: Speller, Sylvia, 6525 AJ, Nijmegen (NL)
(74) Representative: Ketelaars, Maarten F.J.M.

(57) **Abstract**

The invention provides a method for ex vivo analysis of a cell (100). The method includes penetrating the cell membrane (110) of the cell (100) with a first nano electrode (10) and a plurality of second nano electrodes (20, 20', 20", ...), wherein the first nano electrode (10) and the second nano electrodes (20, 20', 20", ...) are longitudinally conductive, and measuring a current, as function of time, between the first nano electrode (10) and one or more second nano electrodes (20, 20', 20", ...), thereby providing an measuring result. The invention further provides an apparatus (1000) for using this method.

## Description

### Field of the invention

The invention relates to a method for the ex vivo analysis of a cell as well as to an apparatus therefore.

### Background of the invention

Nanotechnology has received increased attention in the biological research field. Important examples are the usage of nanoparticles in optical and magnetic resonance imaging, the demonstration of potential application of metal nano shells and carbon nano tubes for the treatment of tumor and cancer cells, and the application of nano wire-based transistors to electrically detect specific biomolecules. In all of these cases, the nano materials are functioning either inside the cells or at the vicinity of the surface ofbiomolecules.

US 2007275433 describes a system with isolated cells comprising nano patch sensors integrated into the cell membrane thereof, wherein said sensors are provided in the form of perturbation-sensitive constructs, and wherein said perturbation-sensitive constructs respond to perturbations of the cell membrane by means of a detectable change in one or more physical or chemical properties associated with said construct.

US 2004186459 describes a method for delivering a fluid into a cell, the method comprising: inserting a first end of a nano tube into the cell, connecting a second end of the nano tube to a fluid supply, and transferring fluid from the fluid supply into the cell via the nano tube. Further, this document describes a fluid delivery device for delivering a fluid to at least one cell, said fluid delivery device comprising: a support having a passageway therethrough, a nano tube disposed in said passageway, said nano tube having a first end extending from a surface of said support, and a sidewall extending from said support to form a container for containing a fluid in fluid communication with a second end of said nano tube. Further, these passageway may comprise a plurality of spaced-apart passageways, said at least one nano tube comprises a plurality of nano tubes each of which being disposed in a different one of said plurality of passageways, and said plurality of nano tubes having a plurality of first ends extending from said surface of said support.

Further, it is stated in the art that carbon nano tube transistors have outstanding potential for electronic detection of biomolecules in solution. The physical mechanism underlying sensing is however reported to remain controversial, which hampers full exploitation of these promising nano sensors.

### Summary of the invention

Sensing the inner membrane volume of a cell may provide information about the type and/or status of such cell, such as the vitality of the cell, oxidative stress present in the cell, trauma history of the cell, irradiation imposed to the cell, toxins to which the cell was exposed, metabolomic parameters of the cell, nutrition level of the cell, and epigenetic imprint of the (stem)cell.

Hence, it is an aspect of the invention to provide an alternative method for the analysis of a cell as well as an apparatus suitable for performing such method.

The invention provides in an embodiment a method for ex vivo analysis of a cell comprising penetrating the cell membrane of the cell with a first nano electrode and a plurality of second nano electrodes, wherein the first nano electrode and the second nano electrodes are longitudinally conductive, and measuring a current as function of time between the first nano electrode and one or more second nano electrodes, thereby providing an measuring result.

Such method may allow mapping of the cell and may allow providing a fingerprint of the cell. By measuring the currents and especially by comparing with, in the same way, fingerprints of well characterized reference cells, information about the cell under investigation from the measuring result may be derived. Hence, in a specific embodiment, the measuring result is further compared with one or more reference measuring results of one or more reference cells; based on this comparison, the cell under investigation can be evaluated. Therefore, in a specific embodiment, the method further comprises comparing the measuring result with reference analysis data of a reference cell, and determining the type and/or status of the cell.

For instance, in this way, well characterized cell can be used to measure a measuring result and define that measuring result as reference measuring result (i.e. reference fingerprint or reference cell mapping). This result can for instance be stored on a data carrier. Learning software, for instance using ANN (Artificial Neural Network) can be used to teach the software to define the reference cell, but may also be used to teach the software to recognize cells for analysis. In this way, a reference library can be created.

In this way, also well characterized cells which have been subjected to well characterized conditions, such as cell aging, stress, or cells which are in a specific defined stadium, or cells that have changed into tumor cells, etc., may be analyzed to provide measuring results, which than may again be used as reference measuring results. Hence, also in this way, learning software may be used to further fill the reference library. All data available in the reference library can be used again to compare with the measuring result of a specific cell under investigation, which may then lead to an evaluation, such as type or status of the cell.

The method and apparatus (see below) according to embodiments of the invention allow such measurements, since the electrodes penetrate into the cell, through the cell membrane, but since the electrodes are nano electrodes, the cell membrane is not irreversibly damaged and the cell stays alive. In contrast, in well known methods from the prior art cells may irreversibly be damaged. In this way, a fingerprint result of the cell under investigation can be provided.

The method (and the apparatus; see below), may especially be used for analysis of one or more of food or tissue, especially in the field of food safety or forensics.

The cell may be prokaryotic or eukaryotic; preferably eukaryotic cell types can be analysed.

The invention also provides an apparatus comprising a first nano electrode and a plurality of second nano electrodes, of which preferably at least one of the first nano electrode or the plurality of second nano electrodes is arranged movable, wherein the first nano electrode and the second nano electrodes are longitudinally conductive, and an measuring unit, arranged to measure a current as function of time between the first nano electrode and the second nano electrodes, thereby arranged to provide an measuring result.

In a specific embodiment, the first nano electrode and the second nano electrodes have diameters in the range of about 5-50 nm, and preferably lengths in the range of about 20-200 nm. This may especially lead to good penetration of the cell membrane, while keeping the cell alive. Further, in yet another embodiment, the first nano electrode has a conductive tip and the second nano electrodes have conductive tips, wherein during measuring, a shortest tip distance between tip of the first nano electrode and the tips of the second nano electrodes is maintained in the range of about 200-1000 nm, especially in the range of about 250-800 nm, such as in the range of about 300-700 nm. The second nano electrodes are preferably arranged to provide the shortest distance between second nano electrode tips also in the range about 200-1000 nm, especially in the range of about 250-800 nm, such as in the range of about 300-700 nm. Using such distances may on the one hand allow a good measuring of currents, while on the other hand may substantially avoid influences of ionic conduction within the cell liquid, which may disturb the signal measured.

The plurality of second nano electrodes may be arranged as regular and as irregular array, and also in a combination of regular and irregular arrangements. Preferably, the second nano electrodes are arranged in a regular arrangement.

In a preferred embodiment, the nano electrodes, especially at least the plurality of second nano electrodes, are comprised by a substrate. Such substrate may for instance be a semi conductor, such as Si. Herein, the phrase "comprised by a substrate" may relate to nano electrodes attached to a surface of the substrate. It may also relate to etched features on a substrate. Such substrate may in an embodiment be used as support for the cell. Part of the total number of electrodes on the substrate may be used to penetrate the cell.

As mentioned above, preferably the electrodes are longitudinally conductive, i.e. the conduction especially appears in a direction along the length axis of the electrodes, and substantially not in other directions. Such longitudinally conductive electrode may provide the best undisturbed signal. Pure carbon nano tubes, such as known in the art, can not substantially straightforwardly electronically be connected perpendicularly to a substrate (surface). This implies that carbon nano tubes as such, are substantially not the probes of choice in this application. However, if combined with other materials, such as metals inside nanotubes or minerals e.g. as core or cladding, such carbon nano tubes might be used. Hence, in a specific embodiment one or more of the first nano electrode and the second nano electrodes have an insulating cladding. In another preferred embodiment, the first nano electrode and/or the second nano electrodes comprise (semi) conductive nano wires, such as silicon nano wires. Yet in another embodiment, the first and/or second nano electrodes preferably comprise black silicon needles as electrodes.

As mentioned above, the second nano electrodes may be arranged in an array, such as in a 1D array (substantially line arrangement) or 2D array. Especially in such arrays, one or more of the nano electrodes may be arranged (and used) as reference electrodes. Therefore, in a specific embodiment, one or more of the second nano electrodes are arranged as reference electrode. In such embodiments, one or more second electrodes may be arranged as reference electrode(s) and one or more second electrodes are used, in combination with the first nano electrode, to measure a current as function of the time with against the one or more reference electrodes. In such embodiments, the first nano electrodes, the second nano electrode(s) and the reference electrode(s) may penetrate the cell membrane. Hence, in such embodiment, also the reference electrode may especially be in contact with intra cellular liquid. However, the reference electrode may also be arranged external from the cell, such as in an liquid which is substantially isotonic to intracellular liquid (see also below). Thus, in embodiments of the method of the invention the reference electrode may penetrate the cell or may not penetrate the cell.

Herein, unless indicated otherwise, the second electrode is used to measure a current between the first and the second electrode, and the optional second electrode(s) which is(are) arranged as reference electrode(s), is (are) further indicated as reference electrode(s). Hence, unless indicated otherwise, the second electrode is not arranged as reference electrode.

Optionally, the current is measured between the first and the second electrode, while a potential difference is imposed by an external source to these electrodes. This may especially be used for triggering biochemical reactions, prevention of adsorption, or mimicking natural transient potential differences in the cell, etc.. It could also used to perform voltammetry in a small voltage window or to improve signal to noise ratios. Hence, in a specific embodiment, the method of the invention may further comprise applying a predetermined voltage to the first nano electrode and the second nano electrode. In a preferred embodiment, no voltage is applied

There are several ways to penetrate the cell membrane with the electrodes. In an embodiment of the method of the invention, penetrating the cell membrane of the cell with the first nano electrode and the second nano electrode comprises arranging the cell on a substrate (or support) and moving one or more of the first nano electrode, the second nano electrode and the substrate relative to one another until both the first nano electrode and the second nano electrodes penetrate the cell membrane of the cell. Hence, (a) the substrate may be moved, (b) the first nano electrode may be moved, (c) the second nano electrodes may be moved or two or three of these may be moved. In a specific embodiment, the plurality of electrodes is used as substrate. Hence, in a specific embodiment, the substrate comprises the second nano electrodes.

Preferably, while measuring, the cell is surrounded by a liquid. Preferably, this liquid is cell-friendly. Further, preferably, this liquid is isotonic to extra cellular or intra cellular liquid. Herein, isotonic may for instance mean isotonic solution is a solution in which the concentration of solutes in the liquid is essentially equal to that of cytosol of the cell placed in that liquid.

As mentioned above, the apparatus and method may especially be used for determining one or more of the type or status of the cell, wherein the status for example includes one or more of the vitality of the cell, oxidative stress present in the cell, trauma history of the cell, irradiation imposed to the cell, toxins present in the cell, metabolism presence in the cell, nutrition level of the cell, and epigenetics of the cell.

The invention further provides the use of electrochemical scanning tunnelling microscopy and voltametry for bio nano electronic signal analysis of an inner membrane volume of a cell for determining one or more of the type or status of the cell.

### Brief description of the drawings

Embodiments of the invention will now be described, by way of example only, with reference to the accompanying schematic drawings in which corresponding reference symbols indicate corresponding parts, and in which:
Figures 1a-1c schematically depict embodiments of the apparatus according to the invention, with an sample cell;
Figures 2a-2b schematically depict in more detail embodiments according to the present invention;
Figure 3 schematically depicts how the method and apparatus of the invention can be used.

### Description of preferred embodiments

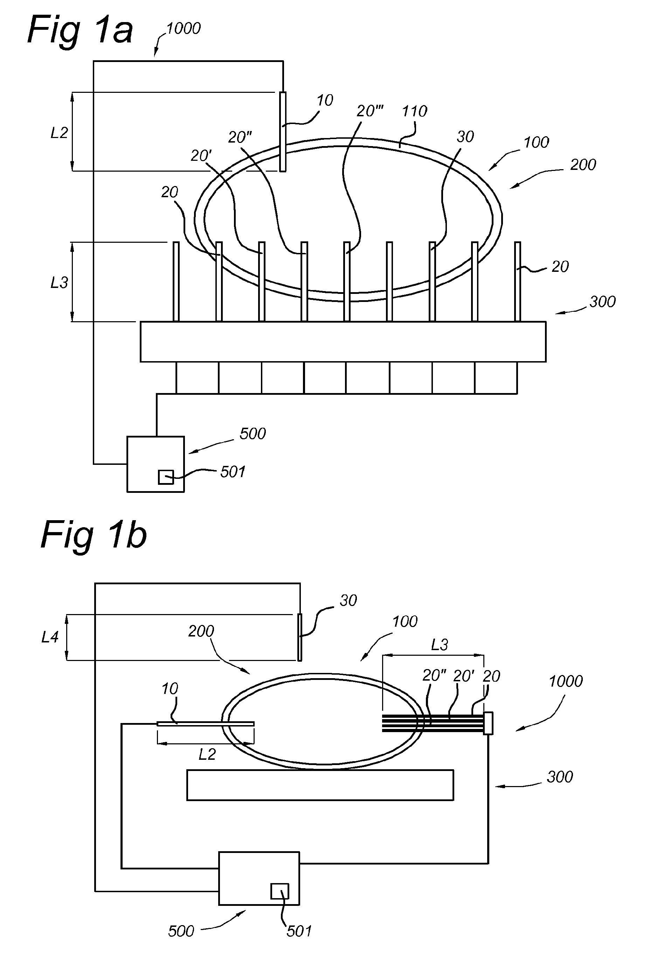

Figure 1a schematically depicts an apparatus 1000, which can also be indicated as intracellular analysis apparatus, or bio-nano electronic analysis apparatus. The apparatus 1000 comprises a first nano electrode 10 (which may optionally comprise a plurality of first nano electrodes) and a plurality of second nano electrodes, indicated with references 20, 20', 20", ... Preferably, at least one of the first nano electrode 10 or the plurality of second nano electrodes 20, 20', 20", ... are arranged movable (see also below), such that they can be used to move and penetrate the cell membrane (indicated with reference 110).

Herein, the first nano electrode 10 is also indicated as first electrode 10 and the second nano electrode 20 is also indicated as second electrode 20. The term "first nano electrode 10" may also relate in an embodiment to a plurality of electrodes. The drawings herein for the sake of understanding only depict one first nano electrode 10.

In this way, a cell 1000, can be penetrated by the first electrode 10 and the second electrodes 20, 20', 20", ... Preferably, the apparatus 1000 comprises 2-400 second electrodes 20, 20', 20", ..., such as 4-50 second electrodes 20. Preferably, the second electrodes are arranged in such a way that the cell 1000 under investigation can be penetrated with at least 2, more preferably at least 4, yet even more preferably at least 8 second electrodes 20.

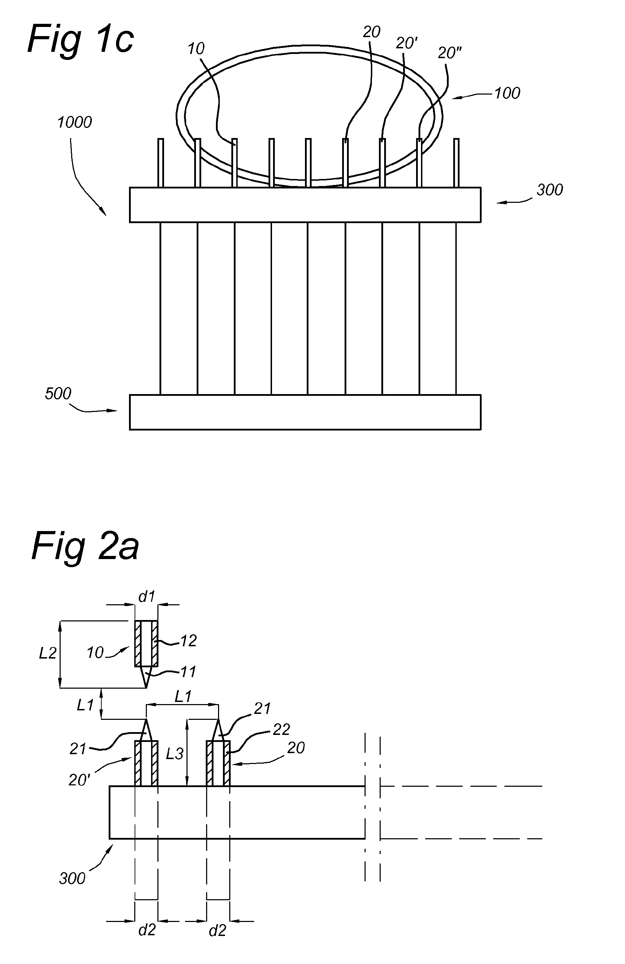

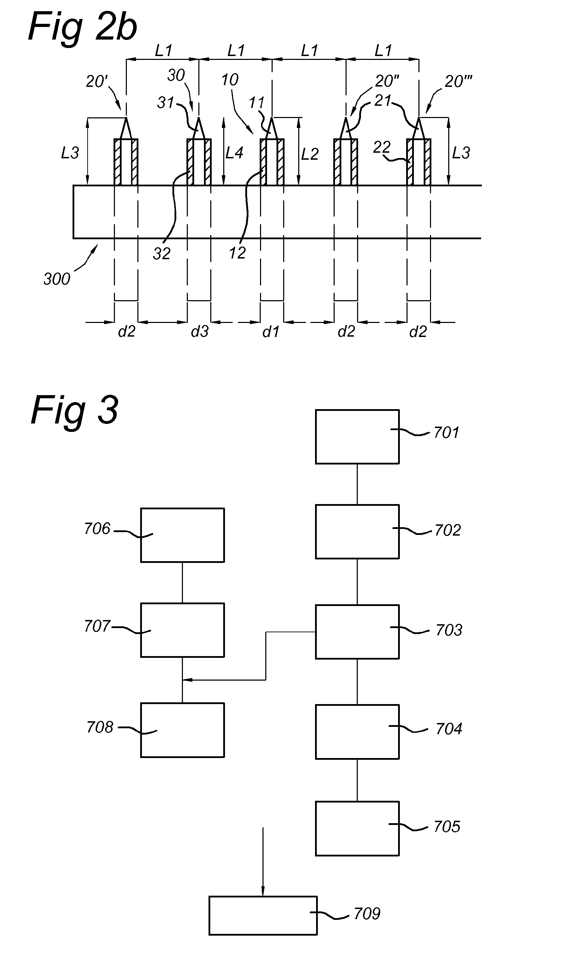

The first nano electrode 10 and the second nano electrodes 20, 20', 20", ... are preferably longitudinally conductive, i.e. conduction takes place in the length direction of the electrodes. Preferably, the first nano electrode 10 and the second nano electrodes 20, 20', 20", ... have diameters in the range of 5-50 nm and lengths in the range of 20-200 nm (see also figure 2). Preferably, the length of the electrodes is at least 50 nm, more preferably at least about 70 nm, since the penetration into the cell 100 is preferably at least about 20 nm, more preferably at least about 40 nm, measured from the cell wall. Therefore, the second electrodes 20, 20', 20"are preferably arranged in such a way that the cell 1000 under investigation can be penetrated for at least about 20 nm, more preferably at least about 50 nm with at least 2, more preferably at least 4, yet even more preferably at least 8 second electrodes 20.

The apparatus 1000 further comprises a measuring unit 500, arranged to measure a current as function of time between the first nano electrode 10 and one or more second nano electrodes 20, 20', 20", .... The apparatus 1000 may read out the current(s) between the first nano electrode 10 and one or more of the second nano electrodes 20, 20'... etc., respectively, which may be done in an embodiment sequentially or in another embodiment parallel. Preferably, the total measuring time, is less than about 2 min, such as less than about 30 seconds, like 1-120 seconds, especially 2-45 seconds, such as about 5-25 seconds. Small currents may be measured, such as in the order of about 0.1-100 pA (pico Ampere). By measuring the current(s) as a function of time, a measuring result is obtained, which is a fingerprint of the cell 1000. The measuring unit 500 will in general comprise a computer readable medium with a library 501 with reference data and/or which can be filled with reference data of reference cells.

In figure 1a, the second electrodes 20, 20', 20", ... are arranged on a substrate 300. This substrate 300 may also be indicated as a socket. In this embodiment, the substrate comprises electrodes (here a plurality of second nano electrodes 20, 20', .. etc). The cell 100 can be pressed (gently) on the substrate 300 comprising the electrodes, thereby, in this embodiment penetrating the cell membrane 110. The cell membrane 110 may have a thickness of about 6 - 12 nm. The cell 100 may have dimensions in the order of about 3-20 µm.

The length of the first nano electrode 10 is indicated with reference L2; the length of the second nano electrodes 20 is indicated with reference L3.

Note that not all second nano electrodes must have the same length. The lengths may differ. Further, the length L2 of the first nano electrode 10 and the lengths L3 of the second nano electrodes 20 may in an embodiment be substantially equal.

In general, the cell 100 will be arranged in a liquid 200, which may be isotonic with the intra cellular liquid of the cell 100.

Figure 1b schematically depicts another embodiment, wherein the cell 100 is arranged on the substrate 300 and the first electrode 10 and/or the second electrodes 20, 20', 20", ... may be arranged to be movable. The cell 100 is thereby "sandwiched" until the electrodes penetrate the cell membrane 110. As will be clear to a person skilled in the art, other configurations are also possible. For instance, assuming the first electrode 10 and the second electrodes 20, 20', 20" being arranged over the substrate 3 00, and the cell 100 being arranged between the substrate and said electrodes 10, 20, 20', 20",..., the electrodes 10, 20, 20', 20",... may be arranged to move in the direction of the substrate 300, or the substrate 300 may be arranged to move in the direction of the electrodes 10, 20, 20', 20",..., or both may be arranged to be movable in the direction of one another, etc. thereby allowing the cell membrane 110 to be penetrated by the electrodes 10, 20, 20', 20", ... In the schematic drawing 1b, the first nano electrode 10 and the second nano electrodes 20, 20', 20", ... are drawn in the state that the electrodes have penetrated the cell 100.

In this embodiment, no electrodes are comprised by the substrate 300. Therefore, in this embodiment the substrate 300 may be purely used as support for cell 100, whereas in embodiments wherein the substrate 300 further comprises electrodes, the substrate 300 may be used as support and as measuring device.

Further, figure 1b schematically depicts a reference electrode 30, relative to which a potential difference between the first electrode 10 and one or more second electrodes 20, 20', 20",..., may be defined. Here, the reference electrode 30 is a separate electrode, arranged in liquid 200. Optionally, this reference electrode 30 may also penetrate the cell 100. This is preferred. The length of the reference electrode 30 is indicated with reference L3.

The length L3 of the reference electrode 30 and/or the material of the reference electrode L3 and/or the diameter of reference electrode 30 may the same as those defined herein for the first nano electrode 10 and the second nano electrodes 20, 20', 20",.... The reference electrode 30 is also a nano electrode. This may also apply to the other herein described embodiments.

Figure 1a showed an embodiment wherein the substrate 300 comprises the second nano electrodes 20, 20', 20", ...; figure 1c again shows such an embodiment wherein the substrate 300 comprises the second nano electrodes 20, 20', 20", ..., but in this embodiment, the substrate 300 also comprises the first electrode 10. For instance, assuming black silicon, or nano wires (see also below), one of the needles of the black silicon or one of the wires of the nano wires may be arranged as first electrode 10; the others may be arranges as second electrodes second 20, 20', 20", ...

In figure c, an array of electrodes is shown, comprising second electrodes 20, 20', 20",..., and the first electrode 10. Thus, in this embodiment, the array of electrodes comprised by substrate 300 comprises the second electrodes 20, 20', 20", ... and the first electrode 10. Especially in such embodiment, the length L2 of the first nano electrode 10 and the lengths L2 of the second nano electrodes 20 may substantially be equal.

Instead of first electrode 10 or in addition to first electrode 10, the array might in an embodiment also comprise the reference electrode 30. Hence, in an embodiment, the substrate 300 may comprise the second electrodes 20, 20', 20",..., and may optionally comprise the first electrode 10 and may optionally comprise the reference electrode 30.

Figure 2a schematically depicts in more detail an embodiment of the electrodes that can be used in the invention, especially the first and the second nano electrodes 10, 20, 20', 20", ...Preferably, the first nano electrode 10 has a conductive tip 11 and the second nano electrodes 20, 20', 20", ... have conductive tips 21, 21', 21",..., wherein a shortest tip distance, indicated with reference L1 between tips 21, 21', 21",... is in the range of 200-1000 nm; i.e. adjacent tips are arranged at a distance of about 200-1000 nm of each other, especially in the range of about 250-800 nm, such as 300-400 nm. The shortest distance L1between the conductive tip 11 of the first electrode 10 and the conductive tips 21 of the second electrodes 20, 20', 20",..., is preferably maintained, during measuring also in the range of about 200-1000 nm, such as about 250-800n, especially 300-400 nm.

Figure 2b schematically depicts an embodiment, wherein the first nano electrode 10, the second nano electrodes 20, 20', 20",..., and the reference electrode 30 are comprises by the substrate. The length and diameter of the reference electrode 30 are indicated with reference L4 and d3, respectively. Note that the length and diameters of all types of electrodes may in principle be substantially the same, respectively. In this embodiment, the reference electrode 30 is indicated to be one of the array of electrodes, and the reference electrode also having a cladding (indicated with reference 32) and a conductive tip (indicated with reference 31).

In the embodiment depicted in figures 2a and 2b, the first nano electrode 10 and the second nano electrodes 20, 20', 20", ... have insulating claddings 12,22, respectively. In this way, longitudinal conduction may be obtained. The first electrode 10 has a diameter d1 and the second electrodes 20, 20', 20",..., have diameters d2. These diameters d1,d2 are not necessarily equal. Further, also the diameters d2 of the different second electrodes 20, 20', 20",..., are not necessarily the same. This also applies to other embodiments than the ones depicted in figures 2a and 2b.

Further, this diameter may vary over the length. Hence, in an embodiment, the diameters refer to mean diameters. Preferably, the maximum diameter of the electrodes 10, 20, 20', 20", especially over the first 20 nm, more preferably over the first 50 nm calculated from the conductive tips 11,21, respectively, is not larger than about 100 nm, especially not larger than about 50 nm. The length of the electrodes is indicated with references L2 and L2' for the first electrode 10 and the second electrodes 20, 20' 20", ..., respectively. Note that the length L2' is not necessarily the same for all electrodes 20, 20' 20", ....

In an embodiment, the second nano electrodes 20, 20', 20", ... comprise semi conductive nano wires. Further, also the first electrode 10 may comprise a semi conductive nano wire. For instance, semi conductive Si nano wires may be used. Examples of Si nano wires (SiNW) are for instance described in JACS 2007 (129), 7228-7229; other type of nano wires (GaP-GaAs) are for instance described in Nano Letters, 2007 (7), pages 3051-3055). In yet another embodiment, the second nano electrodes (20, 20', 20", ...) comprises black silicon needles. In yet another embodiment, the first electrode 10 may also be a black silicon needle. Black silicon is known in the art, such as for instance described in Tsing-Hua Her et al., Applied Physics Letters 73 (12) 1998, 1673-1675.

The electrodes herein are by way of example depicted as regular arrays of electrodes, which are arranged substantially parallel. However, also irregular arrays of electrodes may be applied. Further, if desired, the electrodes may also be arranged under angles relative to each other. These remarks especially apply to the embodiments wherein the substrate comprises the plurality of second nano electrodes 20, 20', 20",..." but may also apply to embodiments wherein the substrate comprises the plurality of second nano electrodes 20, 20', 20",..., and optionally the first electrode 10 and/or optionally the reference electrode 30.

Figure 3 schematically depicts how the method of the invention can be applied. Reference 701 refers to a stage wherein signals of cells from established lines are measured. Such cells are used as reference cells. Subsequently, a data mining correlation in stage 702 may take place. Then, algorithms based on for instance artificial neural networks may be generated in stage 703. Thereby, (reference) fingerprints 704 are generated and a library 705 may be created and optionally refined.

Further, a sample cell may be measured in a measuring stage 706, wherein temporal electronic signals of a sample cell 100 may be measured. A data mining correlation stage 707 may take place and a fingerprint 708 of the cell 100 under investigation may be created. Based on a comparison between the reference fingerprint 705 and the fingerprint 708 of the cell 100 under investigation, an assessment of the type and/or status of the cell 100 may be performed in an analysis or assessment stage 709.

The term "substantially" herein, will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of".

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A method for ex vivo analysis of a cell (100) comprising penetrating the cell membrane (110) of the cell (100) with a first nano electrode (10) and a plurality of second nano electrodes (20, 20', 20", ...), wherein the first nano electrode (10) and the second nano electrodes (20, 20', 20", ...) are longitudinally conductive, and measuring a current, as function of time, between the first nano electrode (10) and one or more second nano electrodes (20, 20', 20", ...), thereby providing an measuring result.

2. The method according to claim 1, wherein the method further comprises comparing the measuring result with reference analysis data of a reference cell to determine the status of the cell (100).

3. The method according to any one of the preceding claims wherein the first nano electrode (10) and the second nano electrodes (20, 20', 20", ...) have diameters (d1,d2) in the range of 5-50 nm, and lengths (L2,L3) in the range of 20-200 nm, and wherein the first nano electrode (10) has a conductive tip (11) and the second nano electrodes (20, 20', 20", ...) have conductive tips (21, 21', 21",...), wherein a shortest tip distance (L1) between tips (11 and 21) is maintained in the range of 200-1000 nm.

4. The method according to any one of the preceding claims, wherein one or more of the first nano electrode (10) and the second nano electrodes (20, 20', 20", ...) have an insulating cladding (12,22).

5. The method according to any one of the preceding claims, wherein the second nano electrodes (20, 20', 20", ...) comprise semi conductive nano wires.

6. The method according to any one of the preceding claims, wherein the second nano electrodes (20, 20', 20", ...) comprise black silicon needles.

7. The method according to any one of the preceding claims, further comprising applying a predetermined voltage to the first nano electrode (10) and the second nano electrode (20, 20', 20", ...);

8. An apparatus (1000) comprising
- a first nano electrode (10) and a plurality of second nano electrodes (20, 20', 20", ...), wherein the first nano electrode (10) and the second nano electrodes (20, 20', 20", ...) are longitudinally conductive; and
- an measuring unit (500), arranged to measure a current as function of time between the first nano electrode (10) and one or more second nano electrodes (20, 20', 20", ...), thereby arranged to provide a measuring result.

9. The apparatus (1000) according to claim 8, wherein the first nano electrode (10) and the second nano electrodes (20, 20', 20", ...) have diameters (d1,d2) in the range of 5-50 nm and lengths (L2,L3) in the range of 20-200 nm.

10. The apparatus (1000) according to any one of claims 8-9, wherein the second nano electrodes (20, 20', 20", ...) have conductive tips (21, 21', 21",...), wherein a shortest tip distance (L1) between tips (21, 21', 21", ...) is in the range of 200-1000 nm.

11. The apparatus (1000) according to any one of claims 8-10, wherein one or more of the first nano electrode (10) and the second nano electrodes (20, 20', 20", ...) have an insulating cladding (12,22).

12. The apparatus (1000) according to any one of claims 8-11, wherein one or more of the first nano electrode (10) and the second nano electrodes (20, 20', 20", ...) comprise semi conductive nano wires.

13. The apparatus (1000) according to any one of claims 8-12, wherein the second nano electrodes (20, 20', 20", ...) comprises black silicon (400) comprising a plurality of needles as electrodes.

14. The apparatus (1000) according to any one of claims 8-13, wherein the substrate (300) comprises the second nano electrodes (20, 20', 20", ...).

15. Use of the apparatus (1000) according to any one of claims 8-14 for determining one or more of the type or status of the cell (100).

16. Use of the apparatus (1000) according to any one of claims 8-14, for analysis of one or more of food or tissue, especially in the field of food safety and forensics.
